# EUROPEAN PATENT APPLICATION

(11) **EP 1 431 379 A1**
(43) Date of publication of application: **23.06.2004**
(21) Application number: 02772941.7
(22) Date of filing: 27.09.2002
(51) Int. Cl.: C11B 1/10, C11C 1/00, A23D 9/00, C07C 62/32, C07C 51/48

(54) **PROCESS FOR PRODUCING FAT COMPOSITIONS CONTAINING OLEANOLIC ACID AND/OR MASLINIC ACID**

(30) Priority: 28.09.2001 JP 2001304731
(71) Applicant: The Nisshin OilliO, Ltd., Tokyo 104-8285 (JP)
(72) Inventor: KUNO, N.; c/o THE NISSHIN OILLIO, LTD., Yokosuka-Shi, Kanagawa 239-0832 (JP); SHINOHARA, G.; c/o THE NISSHIN OILLIO, LTD., Yokosuka-Shi, Kanagawa 239-0832 (JP)
(74) Representative: Bawden, Peter Charles
(86) International application number: PCT/JP2002/010101
(87) International publication number: WO 2003/029391

(57) **Abstract**

The present invention relates to a method for industrially efficiently preparing an oil and fat composition containing oleanolic acid, maslinic acid, physiologically acceptable salts thereof and derivatives thereof, wherein the method comprises the step of extracting olive plants and/or a by-product obtained in an olive oil-manufacturing process with an organic solvent or a water-containing organic solvent.

## Description

### Background of the Invention

The present invention relates to a method for the preparation of an oil and fat composition containing oleanolic acid and/or maslinic acid, physiologically acceptable salts thereof or derivatives thereof starting from olive plants (Olea europaea L.) and/or by-products (or products) obtained in olive oil-manufacturing process.

The olive is a plant belonging to the genus Olive of the family Oleaceae, which has habitually been used as a raw material for foods. More specifically, the olive is a plant, which has long been grown and the representative olive-growing area is, at present, the district along the shore of the Mediterranean. Regarding the applications thereof, the olive oil among others is important not only as an oil stuff, which has been used in not only Europe, but also various countries in the world including Japan and the United States, but also as a food such as salt-preserved olive fruits, a raw material for cosmetics or as a medicinal plant. Moreover, the olive oil cake has been used as raw materials for fertilizers, feeds and fuels. In other words, the olive may be considered to be a plant relatively stably available and may be vegetable raw materials having high safety to the human body.

It has recently been known that the olive oil obtained through the oil expression of the olive plants is one relatively unsusceptible to oxidation, polyphenols included therein as trace components have attracted special interest and there have been conducted various studies to elucidate, for instance, physiological functions thereof (see, for instance, International Olive Oil Council, New Food Industry, 1992, Vol. 34, No. 4, p.28-52).

In addition, there has also been elucidated, for instance, physiological functions of oleanolic acid present in the leaves of olive plants as one of other components of the olive plants.

Oleanolic acid is a kind of oleanane type triterpene and a compound present in, for instance, Japanese green gentian, clove, rinds of grapes and leaves of olive plants in its free acid state and present in, for instance, Panax schinseng Nees var. japonicum Makino, carrot and sugar beet in the form of saponin. This compound is likewise commercially available. Up to this time, a large number of research workers have conducted studies of, for instance, physiological functions of oleanolic acid and there have been known a variety of functions thereof such as a carcinogenic promoter-inhibitory function (Japanese Un-Examined Patent Publication Sho 63-57519); an anti-inflammatory function and a wound-healing promotion function (Japanese Examined Patent Publication Hei 4-26623); an alcohol absorption-inhibitory function (Japanese Un-Examined Patent Publication Hei 7-53385); and a new hair growing promotion function (Japanese Un-Examined Patent Publication Hei 9-157139).

On the other hand, maslinic acid is a kind of oleanane type triterpene and a compound present in, for instance, olive, hop, Japanese mint, pomegranate, clove, sage and jujube, but it is not so widely distributed in the nature. It has been known that the compound has an anti-inflammatory function and an anti-histaminic function.

As has been described above, oleanolic acid and maslinic acid are useful substances, the olive contains the both oleanolic acid and maslinic acid and accordingly, these triterpenes should stably be supplied by, for instance, incorporating them into oils and fats such as olive oil. However, substances such as oleanolic acid and maslinic acid are components present in relatively small amounts and they are present in a low content in the olive oil obtained through compression. Moreover, they are included in the extracted oil derived from olive, but they are in general removed from the extracted oil during the steps for the usual purification of the same.

For this reason, there has not yet been known any effective method, which can prepare an oil and fat composition containing at least one member selected from the group consisting of oleanolic acid and maslinic acid and physiologically acceptable salts thereof and derivatives thereof starting from the same naturally occurring raw material or the olive and which can industrially efficiently produce the composition in large quantities.

### Disclosure of the Invention

It is accordingly an object of the present invention to provide a method for the preparation of an oil and fat composition containing oleanolic acid and maslinic acid, physiologically acceptable salts thereof or derivatives thereof, starting from olive plants and/or by-products obtained in olive oil-manufacturing process and it is also an object of the present invention to provide a method for industrially efficiently preparing an oil and fat composition containing oleanolic acid and maslinic acid, physiologically acceptable salts thereof or derivatives thereof in high contents.

The inventors of this invention have conducted various investigation of the method for the preparation of oil and fat compositions each comprising at least one member selected from the group consisting of oleanolic acid and/or maslinic acid, physiologically acceptable salts thereof and derivatives thereof, starting from olive plants in order to achieve the foregoing object and have found out a method, which can quite efficiently produce the intended oil and fat composition and have thus completed the present invention.

Thus, the present invention provides a method for the preparation of an oil and fat composition comprising at least one member selected from the group consisting of oleanolic acid, maslinic acid, physiologically acceptable salts thereof and derivatives thereof, in which the method comprises the step of extracting olive plants and/or by-products obtained in olive oil-manufacturing process with an organic solvent or a water-containing organic solvent.

According to another aspect of the present invention, there is also provided a method for the preparation of an oil and fat composition comprising at least one member selected from the group consisting of oleanolic acid, maslinic acid and physiologically acceptable salts thereof, wherein the method comprises the step of extracting olive plants and/or by-products obtained in olive oil-manufacturing process with an organic solvent or a water-containing organic solvent.

According to a further aspect of the present invention, there is also provided a method for the preparation of an oil and fat composition comprising at least one member selected from the group consisting of oleanolic acid, maslinic acid, physiologically acceptable salts thereof and derivatives thereof, in which the method comprises the step of extracting olive plants and/or by-products obtained in olive oil-manufacturing process with an organic solvent or a water-containing organic solvent to thus give an oil and fat composition having an oil content ranging from 20 to 60% by mass.

In this connection, oleanolic acid and maslinic acid are substances represented by the following structural formulas (I) and (II) respectively and the term "physiologically acceptable salts thereof" used herein means compounds derived from the group: -COOH present in the structural formulas (I) and (II). The kinds of such salts are not restricted to specific ones inasmuch as they can commonly be used in, for instance, cosmetic products, pharmaceutical compositions or foods and beverages.

In a preferred embodiment, the present invention relates to the foregoing method in which the olive plants is at least one member selected from the group consisting of fruits, seeds, rinds, leaves, stems, germs and/or buds of olive plants, and dried products, pulverized products and defatted products thereof; and the present invention relates to the foregoing method in which the by-product obtained in olive oil-manufacturing process is at least one member selected from the group consisting of compression residue, extraction residue, filter cakes (filtration products), squeezed oil, extracted oil, degummed oil lees, deacidified oil lees, dark oil, waste decoloring (or bleaching) agent, deodorization scum and waxy fractions.

The organic solvent used in the extraction treatment is preferably a hydrophilic organic solvent, in particular, a water-containing hydrophilic organic solvent from the industrial standpoint or because of, for instance, its excellent permeability into the plant's tissues and its excellent extraction efficiency. Moreover, the organic solvent preferably used herein, from the viewpoint of the extraction ability, is one comprising at least one member selected from the group consisting of mixed liquids comprising chloroform, methanol and/or ethanol, pyridine, ethanol, dimethylsulfoxide, ethyl acetate and acetone, which are excellent in the ability to solubilize oleanolic acid and/or maslinic acid.

Moreover, it is suitable from the viewpoint of the extraction efficiency to use water and/or an organic solvent whose temperature is not less than 50°C and preferably not less than 60°C and it is also suitable to conduct the extraction treatment under pressure.

Preferably, the present invention relates to the foregoing preparation method in which the concentration treatment, among the concentration and/or purification treatments for the oil and fat composition, is a treatment for increasing the content of, for instance, oleanolic acid and/or maslinic acid, or at least one member selected from the group consisting of soluble fraction recovery treatments and/or insoluble fraction recovery treatments, which make use of the difference in solubility in water and/or an organic solvent; liquid-liquid partition treatments in water-hydrophobic organic solvents; treatments of recovering and/or removing precipitates generating through cooling; recrystallization, re-precipitation, treatments with active carbon; and normal phase and/or reversed phase chromatography treatments. In addition, the present invention likewise relates to a method for the preparation of an oil and fat composition having a relatively high content of the at least one member selected from the group consisting of oleanolic acid and/or maslinic acid and physiologically acceptable salts thereof and derivatives thereof, in which the foregoing purification treatment is a treatment of removing impurities present in the oil and fat composition or a treatment of purifying the oils and fats to thus make the same suitable for applications such as edible oils.

Further, regarding the purification treatment of an oil and fat composition such as extracted oil, the inventors of this invention have found that oleanolic acid and/or maslinic acid are considerably removed through the steps such as alkali treatments and steam distillation treatments and that the appropriate control of the conditions for these treatments would advantageously permit the development of a method for preparing an oil and fat composition, which never reduces the content of oleanolic acid and/or maslinic acid. In other words, the present invention relates to a method for the preparation of an oil and fat composition in which the content of, for instance, oleanolic acid and/or maslinic acid and physiologically acceptable salts thereof in the resulting purified oil is not reduced or it is maintained at a high level.

More specifically, the present invention provides a method for the preparation of an oil and fat composition, which contains at least one member selected from the group consisting of oleanolic acid, maslinic acid, physiologically acceptable salts thereof and derivatives thereof, wherein the method comprises the steps of extracting olive plants and/or by-products obtained in olive oil production processes with an organic solvent or a water-containing organic solvent and treating the resulting extract with a weak alkali.

The present invention provides a method for the preparation of an oil and fat composition, which contains at least one member selected from the group consisting of oleanolic acid, maslinic acid, physiologically acceptable salts thereof and derivatives thereof, wherein the method comprises the steps of extracting olive plants and/or by-products obtained in olive oil production processes with an organic solvent or a water-containing organic solvent and subjecting the resulting extract to a light steam distillation.

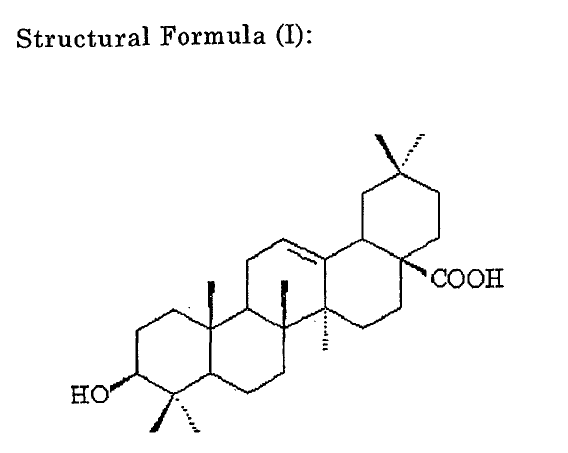

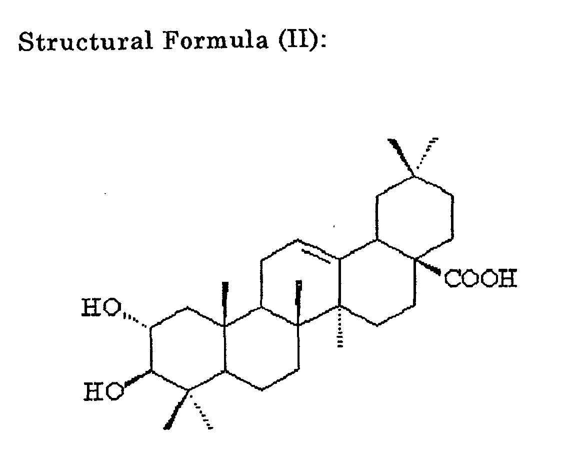

### Best Mode for Carrying Out the Invention

The present invention relates to a method for the preparation of an oil and fat composition containing at least one member selected from the group consisting of oleanolic acid and/or maslinic acid and physiologically acceptable salts thereof and derivatives thereof, wherein the method comprises the step of extracting olive plants and/or by-products obtained in olive oil production processes with an organic solvent or a water-containing organic solvent. In this respect, it is preferred that the method of the present invention is substantially free of any step for the removal of oil fractions. Moreover, it is also preferred that the content of the oil fraction in the oil and fat composition obtained in the extraction step of the preparation method ranges from 20 to 60% by mass. Moreover, the resulting oil and fat composition preferably comprises both oleanolic acid and maslinic acid. Preferably, the present invention relates to a method for the preparation of an oil and fat composition containing at least one member selected from the group consisting of oleanolic acid and/or maslinic acid and physiologically acceptable salts thereof.

In this respect, oleanolic acid and maslinic acid are substances represented by the following structural formulas (I) and (II), respectively. The term "physiologically acceptable salts thereof" used herein means compounds derived from the group: -COOH present in the structural formulas (I) and (II). The kinds of such salts are not restricted to specific ones inasmuch as they can commonly be used in, for instance, cosmetic products, pharmaceutical compositions or foods and beverages.

In this connection, the oil and fat composition of the present invention comprising at least one member selected from the group consisting of oleanolic acid and/or maslinic acid and physiologically acceptable salts thereof and derivatives thereof can be prepared from olive plants; the present invention preferably relates to the foregoing preparation method in which the olive plants is at least one member selected from the group consisting of fruits, seeds, rinds, leaves, stems, germs and/or buds of olive plants, and dried products, pulverized products and defatted products thereof; and the present invention likewise relates to the foregoing method in which the by-product obtained in olive oil-manufacturing process is at least one member selected from the group consisting of compression residue, extraction residue, filter cakes (filtration products), squeezed oil, extracted oil, degummed oil lees, deacidified oil lees, dark oil, waste decoloring (or bleaching) agent, deodorization scum and waxy fractions.

The extraction treatment can be conducted using an organic solvent or a water-containing organic solvent. The extraction treatment may repeatedly be carried out or at least two different extraction methods may be used in combination or at least two extraction treatments using, for instance, different solvents respectively may likewise be used in combination. Extraction treatments and conditions therefor are not restricted to specific ones and the organic solvents usable herein may be both hydrophilic and hydrophobic organic solvents, but the organic solvent used in the extraction treatment is preferably a hydrophilic organic solvent, in particular, a water-containing hydrophilic organic solvent from the industrial standpoint or because of, for instance, its excellent permeability into the plant's tissues and its excellent extraction efficiency. Moreover, the organic solvent preferably used herein, from the viewpoint of the extraction ability, is one comprising at least one member selected from the group consisting of mixed liquids comprising chloroform, methanol and/or ethanol, pyridine, ethanol, dimethylsulfoxide, ethyl acetate and acetone, which are excellent in the ability to solubilize oleanolic acid and/or maslinic acid.

Moreover, it is also suitable from the viewpoint of the extraction efficiency to use an organic solvent or a water-containing organic solvent whose temperature is not less than 50 °C and preferably not less than 60°C since the solubilization ability thereof can be improved and the plant's tissues may suitably get swollen and it is also suitable to conduct the extraction treatment under pressure. Thus, the extraction may further efficiently be carried out.

Preferably, the present invention relates to the foregoing preparation method in which the concentration treatment, among the concentration and/or purification treatments for the oil and fat composition, is a treatment for increasing the content of, for instance, oleanolic acid and/or maslinic acid, or at least one member selected from the group consisting of soluble fraction recovery treatments and/or insoluble fraction recovery treatments, which make use of the difference in solubility in water and/or an organic solvent; liquid-liquid partition treatments in water-hydrophobic organic solvents; treatments of recovering and/or removing precipitates generating through cooling; recrystallization, re-precipitation, treatments with active carbon; and normal phase and/or reversed phase chromatography treatments. In addition, the present invention likewise relates to a method for the preparation of an oil and fat composition having a relatively high content of the at least one member selected from the group consisting of oleanolic acid and/or maslinic acid and physiologically acceptable salts thereof and derivatives thereof, in which the foregoing purification treatment is a treatment of removing impurities present in the oil and fat composition or a treatment of purifying the oils and fats to thus make the same suitable for applications such as edible oils.

Further, regarding the purification treatment of an oil and fat composition such as extracted oil, the inventors of this invention have found that oleanolic acid and/or maslinic acid are considerably removed through the steps such as alkali treatments and steam distillation treatments and that the appropriate control of the conditions for these treatments would advantageously permit the development of a method for preparing an oil and fat composition, which never reduces the content of oleanolic acid and/or maslinic acid. In other words, the present invention relates to a method for the preparation of an oil and fat composition in which the content of, for instance, oleanolic acid and/or maslinic acid and physiologically acceptable salts thereof in the resulting purified oil is not reduced or it is maintained at a high level.

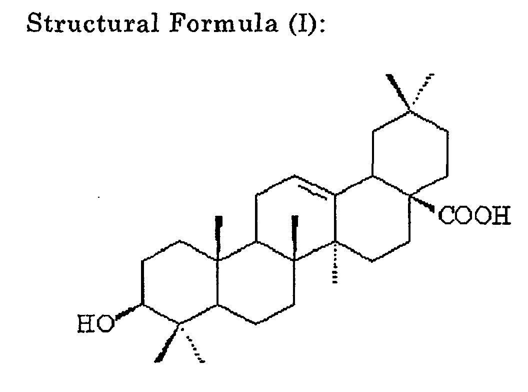

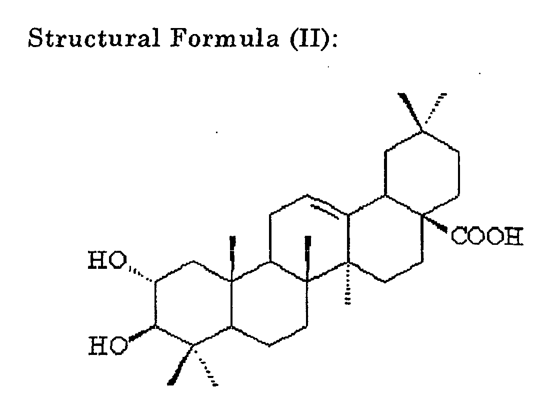

Oleanolic acid and maslinic acid are substances represented by the foregoing structural formulas (I) and (II), respectively.

Oleanolic acid is a compound belonging to the oleanane triterpene and present in, for instance, Japanese green gentian, clove, rinds of grapes and leaves of olive plants in its free acid state and also present in, for instance, Panax schinseng Nees var. japonicum Makino, carrot and sugar beet in the form of saponins. In addition, this compound may easily commercially be available. Oleanolic acid has variously be investigated on, for instance, physiological effects and it has been elucidated that this compound possesses, for instance, a carcinogenic promoter-inhibitory function (Japanese Un-Examined Patent Publication Sho 63-57519), an anti-inflammatory function and a wound-healing promotion function (Japanese Examined Patent Publication Hei 4-26623), an alcohol absorption-inhibitory function (Japanese Un-Examined Patent Publication Hei 7-53385) and a new hair-growing promotion function (Japanese Un-Examined Patent Publication Hei 9-157139).

Maslinic acid is a compound belonging to the oleanane triterpene and present in, for instance, olive, hop, Japanese mint, pomegranate, clove, sage and jujube. It has been known that the compound has an anti-inflammatory function and an anti-histaminic function.

The term "physiologically acceptable salts thereof" used herein means salts derived from the group: -COOH present in the structural formulas (I) and (II). The kinds of such salts are not restricted to specific ones inasmuch as they can commonly be used in, for instance, cosmetic products, pharmaceutical compositions or foods and beverages and specific examples thereof include alkali metal salts such as sodium, potassium and lithium salts; alkaline earth metal salts such as calcium, magnesium, barium and zinc salts; alkylamine salts such as methylamine, dimethylamine, trimethylamine, ethylamine, diethylamine, triethylamine, propylamine, butylamine, tetrabutylamine, pentylamine and hexylamine salts; alkanolamine salts such as ethanolamine, diethanolamine, triethanolamine, propanolamine, dipropanolamine, isopropanolamine and diisopropanolamine salts; salts with other organic amines such as piperazine and piperidine; and salts with basic amino acids such as lysine, alginine, histidine and tryptophane.

The oil and fat composition comprising, for instance, oleanolic acid and/or maslinic acid can be obtained from the fruits, seeds, rinds or seed coats, stems and germs and buds of the olive plant and it is also suitably obtained from the dried products, pulverized products and defatted products thereof.

The olive plant (Olea europaea L.) used in the present invention as a raw material may be any one irrespective of the habitats and may thus be those home-grown or Europe-growth or may be edible ones or those used for the oil expression. The extract used in the invention can be obtained from the fruits, seeds, rinds or seed coats, stems and germs and buds of the olive plant as a naturally occurring plant and the extract may likewise suitably be obtained from the dried products, pulverized products and defatted products of the foregoing raw materials. Among these raw materials, preferably used herein are the dried products, pulverized products and defatted products (compression residue and extraction residue) of olive plants.

Moreover, water is preferably added to the foregoing fruits of the olive plant or the defatted product thereof, or the foregoing fruits or the defatted product thereof are humidified by a steaming treatment. Thus, they appropriately get swollen and therefore, the extraction efficiency is substantially improved.

The defatted product of olive plants also includes substances such as oleanolic acid and/or maslinic acid in high concentrations and it can suitably be used as a raw material therefor. In this regard, the defatted product as a raw material for these compounds may be, for instance, those derived from the compression of olive plants or those obtained after the extraction with, for instance, hexane.

The oil and fat composition of the present invention, which comprises at least one member selected from the group consisting of oleanolic acid and/or maslinic acid and physiologically acceptable salts thereof and derivatives thereof, can be obtained from olive plants through the extraction thereof with an organic solvent or a water- containing organic solvent and the composition preferably comprises, for instance, at least one member selected from the group consisting of oleanolic acid and/or maslinic acid and physiologically acceptable salts thereof.

The extraction treatment can be conducted using an organic solvent or a water-containing organic solvent. The extraction treatment may repeatedly be carried out or at least two different extraction methods may be used in combination or at least two extraction treatments using, for instance, different solvents respectively may likewise be used in combination. Extraction treatments and conditions therefor are not restricted to specific ones. In the present invention, however, the oil and fat composition comprising, for instance, oleanolic acid and/or maslinic acid and physiologically acceptable salts thereof can be obtained by extracting the foregoing raw material with water, an organic solvent or a water-containing organic solvent.

The organic solvents usable in the extraction treatment may be both hydrophilic and hydrophobic organic solvents, but the organic solvent used in the extraction treatment is preferably a hydrophilic organic solvent, in particular, a water-containing hydrophilic organic solvent from the industrial standpoint or because of, for instance, its excellent permeability into the plant's tissues and its excellent extraction efficiency. This is because, the moisture present may get swollen the cells and tissues to thus improve the extraction efficiency and accordingly, the use of a water-containing hydrophilic organic solvent is preferred because of the achievement of a high extraction efficiency or from this standpoint. The organic solvent used in the preparation, from olive plants, of the oil and fat composition of the present invention, which comprises, for instance, oleanolic acid and/or maslinic acid and physiologically acceptable salts thereof may be either a hydrophilic organic solvent or a hydrophobic organic solvent. More specifically, examples of hydrophilic organic solvents used herein include alcohols such as methyl alcohol, ethyl alcohol, glycerin, propylene glycol and 1,3-butylene glycol and known organic solvents such as acetone, tetrahydrofuran, acetonitrile, 1,4-dioxane, pyridine, dimethylsulfoxide, N,N-dimethylformamide and acetic acid; and examples of hydrophobic organic solvents are known organic solvents such as pentane, hexane, heptane, cyclohexane, carbon tetrachloride, chloroform, dichloromethane, 1,2-dichloro- ethane, diethyl ether, ethyl acetate, n-butanol, benzene and toluene. These organic solvents may be used alone or in any combination of at least two of them.

From the industrial standpoint or from the viewpoint of the permeability into the plant's tissues and the extraction efficiency, it is preferred to use a hydrophilic organic solvent and the use of a water-containing organic solvent is preferred. Specific examples thereof are alcohols such as methyl alcohol, ethyl alcohol, glycerin, propylene glycol and 1,3-butylene glycol; organic solvents such as acetone, tetrahydrofuran and acetonitrile; and water-containing ones comprising the foregoing solvents. The oil and fat composition of the present invention, which contains, for instance, oleanolic acid and/or maslinic acid and physiologically acceptable salts thereof can be obtained from the olive plants using at least one member selected from the solvents listed above.

Moreover, the organic solvents used in the extraction may likewise be those having a high solubilizing ability of, for instance, oleanolic acid and/or maslinic acid.

The solvent having a high solubilizing ability herein used means one excellent in its ability to solubilize, for instance, oleanolic acid and/or maslinic acid. Specific examples thereof include one comprising at least one member selected from the group consisting of mixed liquids comprising chloroform, methanol and/or ethanol, pyridine, ethanol, dimethylsulfoxide, ethyl acetate and acetone. These solvents can preferably be used herein since they are excellent in the ability to solubilize oleanolic acid and/or maslinic acid and physiologically acceptable salts thereof and this accordingly permits, for instance, the extraction, from olive plants or the like, of oleanolic acid and/or maslinic acid and physiologically acceptable salts thereof in high concentrations.

The conditions for the extraction are not restricted to specific ones, but the extraction is, for instance, carried out at a temperature ranging from 5 to 95°C, preferably 10 to 90°C and more preferably 15 to 85°C and the extraction can likewise suitably be conducted even at ordinary temperature. It is particularly preferred to conduct the extraction at a temperature ranging from 20 to 80°C. There is observed such a tendency that the extraction efficiency increases as the temperature increases. The extraction can efficiently be carried out at ordinary pressure, under pressure or under reduced pressure established by, for instance, aspiration or suction. The extraction may be carried out according to the shaking extraction or using an extraction device equipped with, for instance a stirring machine. The extraction time may vary depending on other extraction conditions, but it in general ranges from several minutes to several hours and more sufficient extraction may be ensured as the extraction time increases. However, it may appropriately be determined while taking into consideration the production devices used and other production conditions such as yield. For instance, the extraction may be carried out for 0.5 to 10 hours and preferably 1 to 8 hours.

Moreover, the amount of the solvent used in the extraction may be 1 to 100 times (mass/mass, those in the following description are shown in the same way also) and preferably 1 to 20 times that of the raw material used.

Moreover, it is also suitable from the viewpoint of the extraction efficiency to use water and/or an organic solvent whose temperature is not less than 50°C and preferably not less than 60°C since the extraction can suitably be carried out because of the improved solubilization ability thereof and the ability thereof to swell plant's tissues and it is also preferred to conduct the extraction treatment under pressure and this would in turn permit the efficient extraction. Moreover, if taking into consideration factors such as the yield or content of, for instance, oleanolic acid and/or maslinic acid and physiologically acceptable salts thereof in the resulting oil and fat composition, the extraction is preferably carried out using a water-containing lower alcohol whose lower alcohol content is not less than 10% by mass. The extraction is more preferably carried out using a water-containing lower alcohol having a lower alcohol content ranging from 10 to 95% by mass, more preferably 30 to 95% by mass, further particularly preferably 50 to 95% by mass and most preferably 65 to 95% by mass. As such a lower alcohol, ethyl alcohol is particularly preferred.

Examples of alcohols used in the present invention are primary alcohols such as methyl alcohol, ethyl alcohol, 1-propanol and 1-butanol; secondary alcohols such as 2-propanol and 2-butanol; tertiary alcohols such as 2-methyl-2-propanol; and liquid polyhydric alcohols such as ethylene glycol, propylene glycol and 1,3-butylene glycol. These solvents may be used alone or in any combination of at least two of them.

The term "lower alcohol" used herein means a known alcohol having 1 to 4 carbon atoms and examples thereof are primary, secondary tertiary and polyhydric alcohols listed above, which may be used alone or in any combination of at least two of them.

In the preparation method of the invention, it is particularly preferred to use ethyl alcohol, a water-containing ethyl alcohol whose alcohol content ranges from 30 to 95% by mass, ethyl acetate or hexane as the organic solvent or water-containing organic solvent.

The oil and fat composition of the present invention comprising, for instance, oleanolic acid and/or maslinic acid and physiologically acceptable salts thereof can be obtained by the removal of the solvent and/or water from the extract thus prepared.

In this connection, the solvent and/or water can be removed according to any known method such as distillation under reduced pressure and suction and/or vacuum drying techniques.

The use of the extract obtained from defatted products is preferred in the invention since it has high contents of, for instance, oleanoiic acid and/or maslinic acid and physiologically acceptable salts thereof. In addition, the defatted product includes the residue remaining after the oil expression and usable herein as raw materials include the compression residues remaining after the olive oil expression and extraction residues. Therefore, the method is an extremely excellent one, which make the most use of the olive. Moreover, the method employs materials, which are commonly disposed or used in feeds and accordingly, it is also excellent. in the light of the production cost.

As has been described above, the oil and fat composition prepared by the method of the present invention comprises oleanolic acid and/or maslinic acid and physiologically acceptable salts thereof or derivatives thereof and the composition preferably comprises oleanolic acid and/or maslinic acid and physiologically acceptable salts thereof. In this connection, oleanolic acid and/or maslinic acid constitute the major part of the product prepared from olive plants and/or by-products obtained in the olive oil-manufacturing process and therefore, the composition of the present invention more preferably comprises oleanolic acid and/or maslinic acid.

In addition to the foregoing, the oil and fat composition comprising, for instance, oleanolic acid and/or maslinic acid can likewise suitably be prepared from other products obtained in olive oil-manufacturing process such as compression residue, extraction residue, filter cakes (filtration products), squeezed oil, extracted oil, degummed oil lees, deacidified oil lees, dark oil, waste decoloring (or bleaching) agent, deodorization scum and waxy fractions. The raw materials usable in the preparation method of the present invention may be olive plants and/or by-products obtained in olive oil-manufacturing process, with the by-products obtained in olive oil-manufacturing process being preferably used in the preparation method. In particular, raw materials preferably used herein include, for instance, compression residue, extraction residue, deodorization scum and waxy fractions.

The present invention relates to a method for industrially efficiently producing an oil and fat composition comprising at least one member selected from the group consisting of oleanolic acid and/or maslinic acid and physiologically acceptable salts and derivatives thereof, starting from olive plants and/or by-products obtained in olive oil-manufacturing process and preferably to a method for industrially efficiently producing an oil and fat composition comprising at least one member selected from the group consisting of oleanolic acid and/or maslinic acid and physiologically acceptable salts.

The compression residue is obtained when compressing olive plants, in particular, fruits and feeds thereof and it is preferably used in the method of the invention since it comprises large amounts of oleanolic acid and/or maslinic acid and physiologically acceptable salts thereof, which are not dissolved in the squeezed oil or liquate out into the oil. If it has a high moisture content, it is liable to undergo putrefaction and therefore, it is often dried. In addition, the compression residue is preferably in the form of pulverized and/or compressed flat pieces. This compression residue comprises a large amount of remaining oil fraction and it may thus be used as a raw material for the extracted oil. More specifically, the compression residue is extracted with a lipophilic organic solvent such as hexane to give extracted oil and the extraction residue is generated during this process. The extraction oil contains a large amount of, for instance, oleanolic acid and/or maslinic acid as compared with the squeezed oil and can thus be preferably used in the invention. Moreover, the extraction residue may preferably be used as a raw material because of its high content of, for instance, oleanolic acid and/or maslinic acid and physiologically acceptable salts thereof.

As edible olive oil products, particularly preferably used are oil products free of any purification or refining step such as high quality squeezed oil (sometimes referred to as extra-virgin or virgin oil). On the other hand, the low quality squeezed oil (sometimes referred to as, for instance, lampante virgin) and extracted oil (sometimes referred to as pomace oil) products are purified before the practical use thereof and by-products are obtained in the purification steps. Examples of oil-refining processes or steps are filtration processes for unpurified oil (a crude oil product) and/or oil free of any deacidifying treatment, degumming processes, deacidifying processes, decoloring processes and deodorization processes and these processes generate, as by-products, filter cakes (filtration products), degummed oil lees, deacidified oil lees, waste decoloring (or bleaching) agent and deodorization scum, respectively.

The filtration process herein used means a step for passing oils and fats containing precipitates prior to the treatment through a filter medium and a filtration product is generated during this step. The oils and fats to be processed according to this filtration are preferably unpurified oil, degummed oil and oil free of any deacidification. In this respect, any usual filter medium can be used and specific examples thereof include Celite.

The degumming step is one, which comprises the steps of adding an appropriate amount of water to oil, heating with stirring and removing purified gum components suspended through, for instance, hydration using a centrifugal machine and degummed oil lees are obtained in this step as a by-product.

The deacidifying step is one in which degummed oil is heated, with stirring, in the presence of an alkaline aqueous solution to convert free acids present therein mainly comprising, for instance, free fatty acids into their salts (soap in case of fatty acids) and to thus remove the same and the resulting product is washed with water. Deacidified oil lees are obtained in this step as by-products.

The alkali usable herein is preferably the usual alkali (basic) substance and more preferably the usual strong alkali substance. The strong alkali means one whose degree of ionization observed when dissolved in water is 1 or almost equal to 1 and examples thereof include caustic soda (sodium hydroxide).

Thus, deacidified oil lees can suitably be obtained from, for instance, squeezed oil and extracted oil and more preferably extracted oil.

This deacidifying process using an alkali and more preferably a strong alkali would permit the removal of about 90% of oleanolic acid and/or maslinic acid and physiologically acceptable salts thereof present in the oil and fat composition. In other words, most of these substances are transferred into the resulting deacidifying oil lees and therefore, oleanolic acid and/or maslinic acid and physiologically acceptable salts thereof can, for instance, be recovered from the deacidified oil lees thus obtained in high efficiency. Thus, the use of such deacidified oil lees is quite preferred since they would permit the preparation of an oil and fat composition containing these components.

The decoloring or bleaching process means one in which oil and a decoloring agent (such as china clay) are admixed together, the resulting mixture is heated with stirring under reduced pressure and it is then filtered to give decolorized oil tinted with an acceptable pale color and this step provides, as a by-product, the waste decoloring agent on which oleanolic acid and/or maslinic acid and physiologically acceptable salts thereof are, for instance, adsorbed.

Oils and fats obtained in any step may be treated in this decoloring step, but the subjects preferably used in this step include, for instance, unpurified oil, oil free of any deacidifying treatment, oil treated with an weak alkali and oil lightly treated by steam distillation because of their high contents of, for instance, oleanolic acid and/or maslinic acid and physiologically acceptable salts thereof. Examples of decoloring agents preferably used herein are activated china clay and activated charcoal.

In this respect, the oil treated with a weak alkali means oils and fats obtained through a treatment with a weak alkali.

The treatment with a weak alkali is one in which a weak alkali is used instead of a strong alkali in the deacidifying treatment using an alkali. In this respect, weak alkalis suitably used herein may be the usual weak alkaline (weakly basic) substances. The weak alkali in general means an alkali whose degree of ionization observed when it is dissolved in water is considerably smaller than 1 and specific examples include those having a degree of ionization as determined at a concentration of 0.1 mole/L on the order of not more than 0.1. Alternatively, examples of weak alkalis are substances capable of receiving a proton from water or other substances. Specific examples thereof cannot unconditionally be listed, but examples of the former include salts of weak acids with strong alkalis (strong bases) such as sodium carbonate, sodium acetate and sodium hydrogen carbonate. On the other hand, a specific example of the latter includes ammonia. The pH value in the deacidification with a weak alkali preferably ranges from 7.5 to 10. The use of a pH value falling within the range specified above is preferred since phenolic substances is not removed from the crude (unpurified) oil in the form of alkaline deacidified oil lees and certainly remain therein, while substances such as free fatty acids can effectively be removed to thus improve the degree of purification of the resulting product. The concentration of such a weak alkali preferably ranges from 1.2 to 1.5 time that of a conventional strong alkali such as caustic soda (sodium hydroxide) when using, for instance, sodium carbonate.

This weak alkaline treatment may preferably be used herein since most of, for instance, oleanolic acid and/or maslinic acid are not transferred to the deacidified oil lees, but remain in the resulting oil and fat composition or this weak alkaline treatment may enhance the content of, for instance, oleanolic acid and/or maslinic acid and physiologically acceptable salts thereof in the resulting composition.

Moreover, the lightly steam-distillated oil means oils and fats obtained through a light steam distillation treatment.

The light steam distillation treatment herein used means a treatment in which the steam distillation carried out in, for instance, a deodorization treatment is conducted under relatively mild conditions and a treating method for simply removing free fatty acids as impurities and volatile component giving out bad smells present in the oil as a subject.

The conditions for the light steam distillation treatment cannot unconditionally be determined, but the degree of vacuum, for instance, ranges from 1.33 × 10² to 1.33 × 10³ Pa, preferably 1.33 × 10² to 9.31 × 10² Pa, more preferably 1.33 × 10² to 6.65 × 10² Pa and further preferably 1.33 × 10² to 3.99 × 10² Pa; the temperature ranges from 140 to 240°C, preferably 140 to 220°C, more preferably 160 to 220°C, further preferably 160 to 200°C and most preferably 180 to 200°C; the treating time ranges from 10 to 120 minutes, preferably 10 to 90 minutes, more preferably 10 to 60 minutes and further preferably 20 to 60 minutes; and the quantity of steam used ranges from 1 to 10% (wt/wt), preferably 1 to 8% and more preferably 1 to 6% per unit mass of the oil to be processed. The light steam distillation treatment is preferably conducted under the foregoing conditions since most of, for instance, oleanolic acid and/or maslinic acid are not transferred to the deodorization scum, but remain in the resulting oil. and fat composition or the treatment conducted under such conditions may enhance the content of, for instance, oleanolic acid and/or maslinic acid and physiologically acceptable salts thereof in the resulting composition.

Moreover, it is sufficient to subject the raw material to this light steam distillation treatment over a relatively short period of time to remove only free fatty acids and the treatment should be continued over a relatively long period of time to remove even the components giving out bad smells, but the treating time may appropriately be controlled while taking into consideration the degree of deterioration of the oil and fat composition to be treated.

The deodorization step herein used means one in which the decolorized oil is steam-distilled under reduced pressure to thus remove the volatile components giving out bad smells present in the oil and the step generates deodorization scum as a by-product. This deodorization step is preferred since not less than 50% by mass of, for instance, oleanolic acid and/or maslinic acid and physiologically acceptable salts thereof present in the oil prior to the deodorization step are transferred to the resulting deodorization scum.

In this connection, oils and fats obtained in any process can be treated in this deodorization step, but the subjects preferably used in this step include, for instance, unpurified (crude) oil, oil free of any deacidifying treatment, oil treated with an weak alkali and oil lightly treated by steam distillation because of their high contents of, for instance, oleanolic acid and/or maslinic acid and physiologically acceptable salts thereof. Examples of devices to be used herein include the usual distillation devices and thin layer distillation devices.

The usual conditions for the steam distillation, used as such a deodorization treatment, are as follows: the degree of vacuum ranges from 13.3 to 1.33 × 10³ Pa, preferably 13.3 to 9.31 × 10² Pa, more preferably 1.33 × 10² to 6.65 × 10² Pa and most preferably 1.33 × 10² to 3.99 × 10² Pa; the temperature ranges from 180 to 260°C, preferably 200 to 260°C, more preferably 220 to 260°C and further preferably 240 to 260°C; the treating time ranges from 10 to 120 minutes, preferably 30 to 120 minutes, more preferably 60 to 120 minutes and further preferably 90 to 120 minutes; and the quantity of steam used ranges from 1 to 10% (wt/wt), preferably 1 to 8% and more preferably 1 to 6% per unit mass of the oil to be processed. The treatment is preferably conducted under the foregoing conditions since the resulting deodorization scum has a higher content of, for instance, oleanolic acid and/or maslinic acid and physiologically acceptable salts thereof.

In this respect, deodorization scum having a higher content of, for instance, oleanolic acid and/or maslinic acid and physiologically acceptable salts thereof can be obtained by conducting the deodorization at a higher degree of vacuum, a higher temperature over a longer period of time, but the conditions therefor may appropriately be selected while taking into consideration various factors such as the equipment used and production cost.

A distillation treatment in which any steam is not used may suitably be employed. In this case, however, it is preferred to use a higher degree of vacuum. For instance, the distillation under a high vacuum such as the molecular distillation permits the preparation of, for instance, oleanolic acid and/or maslinic acid and physiologically acceptable salts thereof.

Among these raw materials, the deacidified oil lees as by-products of the foregoing deacidification step comprise a large amount of a soap fraction and accordingly, they are also used, in particular, as a raw material for fatty acids. A strong acid such as sulfuric acid is added to the deacidified oil lees, the resulting mixture is boiled to thus decompose the soap fraction and the moisture of the product is removed or separated to give dark oil having a high content of free acids such as free fatty acids. In the dark oil derived from the deacidified oil lees having a high content of, for instance, oleanolic acid and/or maslinic acid and physiologically acceptable salts thereof, oleanolic acid and/or maslinic acid are further concentrated. Accordingly, the dark oil is preferably used herein.

The dewaxing step is also referred to as wintering and a step in which high melting point components present in oil are crystallized or precipitated through cooling to thus remove the same and a waxy fraction is obtained as a by-product. The dewaxing treatment is preferred since when oil is sufficiently treated in this dewaxing treatment, oleanolic acid and/or maslinic acid and physiologically acceptable salts thereof can, for instance, be separated from the oil as a waxy fraction.

In this connection, oils and fats obtained in any process can be treated in this dewaxing step, but the subjects preferably used in this step include, for instance, unpurified (crude) oil, oil free of any deacidifying treatment, oil treated with an weak alkali and oil lightly treated by steam distillation because of their high contents of, for instance, oleanolic acid and/or maslinic acid and physiologically acceptable salts thereof.

Regarding the conditions for the dewaxing step, the temperature is not more than 40°C, preferably not more than 30°C, more preferably not more than 20°C, further preferably not more than 15°C, still further preferably not more than 10°C and most preferably not more than 5°C; and the treating time is not less than one minute, preferably mot less than 10 minutes, more preferably not less than 30 minutes, further preferably not less than one hour, still further preferably not less than 3 hours and most preferably not less than 5 hours. The dewaxing treatment is preferably carried out under the foregoing conditions since the resulting waxy fraction has a higher content of, for instance, oleanolic acid and/or maslinic acid and physiologically acceptable salts thereof.

In this respect, a waxy fraction having a higher content of, for instance, oleanolic acid and/or maslinic acid and physiologically acceptable salts thereof can be obtained by conducting the dewaxing step at a lower temperature over a longer period of time, but the conditions for the step may appropriately be selected while taking into consideration various factors such as the equipment used and production cost. In addition, the resulting waxy fraction may be filtered according to the usual method, but a filter aid may, if needed, be used.

In the preparation method of the invention, the oil and fat composition thus prepared may suitably be used without any pretreatment, but the composition is preferably subjected to concentration and/or purification treatments.

The concentration permits the increase in the content of, for instance, oleanolic acid and/or maslinic acid, while the purification permits the removal of impurities present in the oil and fat composition and the purification of oils and fats such that they can suitably be applied to edible oil or used in other applications. Thus, these treatments are preferred since they permit the preparation of an oil and fat composition having a higher content of, for instance, oleanolic acid and/or maslinic acid and a high degree of purification.

The present invention permits the preparation of an oil and fat composition containing, for instance, oleanolic acid and/or maslinic acid through the reuse of by-products obtained in olive oil production processes and usually disposed such as filtration products, degummed oil lees, deacidified oil lees, waste decoloring agents, deodorization scum, dark oil and waxy fractions and further the resulting composition can be concentrated and/or purified to give a composition having a higher content of, for instance, oleanolic acid and/or maslinic acid and accordingly, the method of the present invention is quite excellent from the viewpoint of the production cost and from the industrial standpoint.

The foregoing concentration treatment may be any one insofar as it can improve the content of, for instance, oleanolic acid and/or maslinic acid and physiologically acceptable salts thereof present in the oil and fat composition, but the concentration may suitably be carried out by at least one treatment selected from the group consisting of soluble fraction recovery treatments and/or insoluble fraction recovery treatments, which make use of the difference in solubility in water and/or an organic solvent; liquid-liquid partition treatments in water-hydrophobic organic solvents; treatments of recovering and/or removing precipitates generating through cooling; recrystallization, re-precipitation, treatments with active charcoal; and normal phase and/or reversed phase chromatography treatments. Moreover, the concentration treatment can be repeated or different concentration treatments may be used in combination, but these treatments may properly be selected or determined depending on various factors such as the equipment used and the production cost.

Conditions for the concentration of, for instance, oleanolic acid and/or maslinic acid in the oil and fat composition are not restricted to specific ones. The concentration may, for instance, be carried out by a method, which makes use of the solubility difference in water. Oleanolic acid and/or maslinic acid or the like used in the present invention are compounds having a relatively low polarity and hardly soluble in water. The extract from olive plants can be divided into hardly water-soluble components and/or water-insoluble components or hardly water-soluble or insoluble components and water-soluble components to thus significantly concentrate the extract for the foregoing components, while making use of the foregoing characteristic properties thereof.

The hardly water-soluble or insoluble components can easily be obtained by adding the extract from olive plants in water with stirring and collecting the precipitated fraction through, for instance, filtration.

Alternatively, components present in the oil and fat composition such as oleanolic acid and/or maslinic acid can, if needed, be concentrated according to the usual liquid- liquid partition using a combination of solvents. Such combination of solvents cannot unconditionally be determined, but examples thereof are combinations of water and hydrophobic organic solvents and specific examples of such hydrophobic organic solvents include known ones such as pentane, hexane, heptane, cyclohexane, carbon tetrachloride, chloroform, dichloromethane, 1,2-dichloroethane, diethyl ether, ethyl acetate, n-butanol, benzene and toluene.

The components such as oleanolic acid and/or maslinic acid are hardly soluble in water and therefore, unnecessary water-soluble components can be removed by the separation of the hydrophobic organic solvent phase. In this case, the removal of the solvent would easily permit the recovery of an oil and fat composition having an enhanced content of the components such as oleanolic acid and/or maslinic acid.

The foregoing purification treatment is not limited to any particular one inasmuch as it can improve the content of the components such as oleanolic acid and/or maslinic acid and physiologically acceptable salts thereof present in the oil and fat composition through the removal of impurities present therein and/or the purification of oils and fats in such a manner that they are adapted for use in edible oil or other applications, but subjects such as oils and fats, in particular, extracted oil may suitably be purified by at least one process selected from the group consisting of, for instance, filtration steps for crude oil and/or oil free of any deacidification, degumming steps, deacidifying steps, decoloring steps and deodorizing steps. Moreover, the purification treatment can be repeated or different purification treatments may be used in combination, but these treatments may properly be selected or determined depending on various factors such as the equipment used and the production cost.

Conditions for the concentration of the components such as oleanolic acid and/or maslinic acid in the oil and fat composition are not restricted to specific ones. For instance, the extracted oil as a kind of oil and fat composition can be concentrated by conducting the foregoing purification step. In this connection, by-products such as filtration products, degummed oil lees, deacidified oil lees, waste decoloring agents and deodorization scum are obtained in these steps and these by-products are oil and fat compositions having a high content of at least one member selected from the group consisting of oleanolic acid and/or maslinic acid and physiologically acceptable salts thereof and derivatives thereof. Accordingly, they can preferably be used in the invention.

These concentration treatments may be repeated and similarly, the purification treatments may be repeated. Alternatively, different concentration treatments may be combined and similarly, different purification treatments may be used in combination. Further, a concentration treatment may be followed by a purification treatment, a purification treatment may be followed by another purification treatment, or concentration, purification and additional concentration treatments may be carried out in this order. It is a matter of course that the present invention may comprise combinations of these treatments other than those listed above.

Oil products such as squeezed oil and extracted oil can be subjected to various purification treatments such as filtration steps for crude oil and/or oil free of any deacidification, degumming steps, deacidifying steps, decoloring steps and deodorizing steps to thus remove impurities and to give purified oil suitable for foods, but the useful components such as oleanolic acid and/or maslinic acid are also removed by these purification steps and therefore, the resulting oils and fats have a quite low content of the useful components such as oleanolic acid and/or maslinic acid or they are almost free of any such component. However, the inventors of this invention have found out a means for obtaining an oil and fat composition such as purified (or refined) oil whose content of the useful components such as oleanolic acid and/or maslinic acid is not reduced or it is maintained at a high level by appropriately controlling the conditions for each purification step and the degree of each treatment for oils and fats.

In this respect, usable in the present invention as the oil and fat composition include, for instance, squeezed oil obtained by compressing olive plants and/or the by-products obtained in the olive oil-manufacturing process or extracted oil obtained through the extraction treatment of these raw materials.

Moreover, the control of the processing conditions can suitably realized by the adjustment of those for filtration steps for crude oil and/or oil free of any deacidification, degumming steps, deacidifying steps, decoloring steps and deodorizing steps. Further, the inventors of this invention have found that an alkali treatment and/or a steam-distillation treatment permit the considerable removal of, for instance, oleanolic acid and/or maslinic acid and as a result, they have completed a method for the preparation of an oil and fat composition such as refined oil whose content of the useful components such as oleanolic acid and/or maslinic acid is not reduced or it is maintained at a high level by appropriately controlling the conditions for these processes.

An example of the alkali treatment whose conditions are controlled is a weak alkali treatment. Moreover, an example of the steam-distillation treatment whose conditions are controlled is a light steam-distillation treatment.

In the process for the purification of oils and fats, the combination of these weak alkali treatments and/or light steam-distillation treatments are not restricted to specific ones. Therefore, a weak alkali treatment may be substituted for only the deacidifying step, a light steam-distillation treatment may be substituted for a deodorization step and a light steam-distillation treatment may be substituted for both deacidifying and deodorization steps, but these treatments may arbitrarily be selected or combined while taking into consideration various factors such as the kinds of free fatty acids to be removed, the amounts of components giving out bad smells and the extent thereof.

Moreover, the light steam-distillation treatment may be conducted over a relatively short period of time to remove only the free fatty acids while the treatment should be continued over a relatively long period of time to remove even the components giving out bad smells. However, the treating time may appropriately be controlled while taking into consideration the degree of deterioration of the oil and fat composition to be processed.

In the method for the preparation of the oil and fat composition of the invention, the purification of oils and fats using such a combination of these weak alkali and/or light steam-distillation treatments would permit the preparation of an oil and fat composition such as refined oil whose content of the useful components such as oleanolic acid and/or maslinic acid is not reduced or it is maintained at a high level. More specifically, the method of the present invention permits the preparation of an oil and fat composition comprising at least one member selected from the group consisting of oleanolic acid and/or maslinic acid and physiologically acceptable salts thereof and derivatives thereof or preferably comprising oleanolic acid and maslinic acid, in an amount preferably ranging from 1 to 60% by mass, more preferably 3 to 50% by mass and further preferably 5 to 40% by mass.

Regarding the physiological functions of oleanolic acid and maslinic acid included in the oil and fat composition prepared by the method of the invention, it has been known that oleanolic acid possesses, for instance, a carcinogenic promoter-inhibitory function, an anti-inflammatory function, a wound-healing promotion function, an alcohol-absorption-inhibitory function and a new hair-growing promotion function and that maslinic acid possesses, for instance, an anti-inflammatory function and anti-histaminic function. In addition, maslinic acid also has a bleaching function and an antitumor function.

The oil and fat composition comprising oleanolic acid and maslinic acid possessing such functions can be used in products orally or parenterally administered to human body as will be detailed below as well as other various fields and applications such as feeds for various animals such as domestic animals and fishes, agricultural chemicals and industrial products and other conditions such as their shapes are not restricted to specific ones.

Regarding the oral applications, the composition can be incorporated into, for instance, foods and beverages and orally administered pharmaceutical agents. Specific examples of foods and beverages are diets for treating, health foods and nutritive foods. In this respect, the use of the composition in a diet for treating would permit the prevention of geriatric diseases.

Moreover, the kinds of foods and beverages are not restricted to specific ones, but specific examples thereof are keepable foods, perishable foods, processed marine products, beverages, seasonings, oil and fat foods and dairy products.

In addition, particularly preferred parenteral applications of the composition are, for instance, incorporation thereof into agents externally applied to the skin. The shapes of the agents externally applied to the skin are not restricted to specific ones and examples thereof include pharmaceutical agents, quasi-drugs and cosmetic products. When the composition is, for instance, incorporated into a pharmaceutical agent, the resulting agent may be used as an agent externally applied to the skin having healing and/or improving effects for a variety of disorders of the skin and when it is used as a cosmetic product, it may be used as a medicated cosmetic product, which may have a beautifying effect.

### Examples

Then the present invention will be described in more detail with reference to the following Examples, but the present invention is not restricted to these specific Examples at all. The term "% by mass" appearing in the following description will sometimes referred to as simply "%".

### Example 1

To 1 kg of extraction residue obtained in the olive oil-manufacturing process and indigenous to Italy, there was added 10 volumes of water containing ethanol having an ethanol content of 65% by mass and the resulting mixture was vigorously stirred at 60°C for 4 hours to thus conduct the extraction thereof. The whole mixture was passed through a filter and the resulting filtrate was concentrated to dryness to thus give 52.0 g of an oil and fat composition.

The resulting oil and fat composition and the concentrated product thereof were inspected for the contents of oleanolic acid and maslinic acid present therein according to the capillary GC (gas chromatography) technique. The results thus obtained are listed in the following Table 1.

### Reference Example 1

Water (1040 g) was added to the oil and fat composition prepared in Example 1 and the resulting mixture was vigorously stirred at room temperature. The whole system was subjected to centrifugal separation, the resulting supernatant was removed through decantation and the remaining precipitated fraction was dried to thus give 25.2 g of a concentrated oil and fat composition.

The resulting oil and fat composition and the concentrated product thereof were inspected for the contents of oleanolic acid and maslinic acid present therein according to the capillary GC (gas chromatography) technique. The results thus obtained are listed in the following Table 1.

### Example 2

To 1 kg of extraction residue obtained in the olive oil-manufacturing process and indigenous to Italy, there was added 10 volumes of ethanol and the resulting mixture was vigorously stirred at 60°C for 4 hours to thus conduct the extraction thereof. The whole mixture or system was passed through a filter and the resulting filtrate was concentrated to dryness to thus give 35.8 g of an oil and fat composition.

The resulting oil and fat composition was inspected for the contents of oleanolic acid and maslinic acid present therein according to the capillary GC technique. Moreover, a part of the resulting oil and fat composition was extracted with hexane to thus determine the content of the oil components. The results thus obtained are listed in the following Table 1.

### Example 3

To 1 kg of extraction residue obtained in the olive oil-manufacturing process and indigenous to Italy, there was added 10 volumes of ethyl acetate and the resulting mixture was vigorously stirred at 60°C for 4 hours to thus conduct the extraction thereof. The whole system was passed through a filter and the resulting filtrate was concentrated to dryness to thus give 37.4 g of an oil and fat composition.

The resulting oil and fat composition was inspected for the contents of oleanolic acid and maslinic acid present therein according to the capillary GC technique. Moreover, a part of the resulting oil and fat composition was extracted with hexane to thus determine the content of the oil components. The results thus obtained are listed in the following Table 1.

### Example 4

To 100 kg of compression residue, there was added 10 volumes of hexane, the resulting mixture was vigorously stirred at 60°C for 4 hours to thus conduct the extraction, miscella was filtered at a temperature ranging from 40 to 60°C to remove the impurities and the resulting filtrate was subjected to the removal of the hexane to thus give 12.1 kg of an oil and fat composition.

### Example 5

To 10 kg of the oil and fat composition prepared in Example 4, there was added 0.2 kg of china clay and the former was decolorized at 120 °C for 15 minutes under reduced pressure. The resulting decolorized oil was subjected to a light steam-distillation treatment at a vacuum degree of 3.99 × 10² Pa, a temperature of 190°C, an amount of injected steam of 4% by mass relative to the oil over 60 minutes to thus give 8.8 kg of oil purified by light steam-distillation.

The resulting purified oil was inspected for the contents of oleanolic acid and maslinic acid present therein according to the capillary GC technique. As a result, the oleanolic acid and maslinic acid contents were found to be 4120 ppm and 810 ppm, respectively.

### Reference Example 2

To 10 kg of unpurified oil (crude oil) prepared according to the same procedures used in Example 4, there was added 0.9 kg of a 24% caustic soda (sodium hydroxide) aqueous solution, followed by deacidification at 85°C for 20 minutes with stirring, washing with water, centrifugation and drying to give deacidified oil. In addition, deacidified oil lees were also obtained in this deacidifying step.

Active china clay was added to the resulting deacidified oil in an amount of 2% by mass relative to the oil and the mixture was allowed to stand at 120°C under reduced pressure for 15 minutes for the decoloration of the oil. The resulting decolored oil was deodorized at a degree of vacuum of 3.99 × 10² Pa, a temperature of 250°C and an injected amount of steam of 4% by mass with respect to the oil over 60 minutes to obtain 8.6 kg of purified oil.

The resulting purified oil was inspected for the contents of oleanolic acid and maslinic acid present therein according to the capillary GC technique. As a result, the oleanolic acid and maslinic acid contents were found to be not more than 10 ppm and not more than 10 ppm, respectively.

### Example 6

To deacidified oil lees (the deacidified oil lees derived from extracted oil) obtained in olive oil-manufacturing process (1 kg per dried solid content), there was added a 15% sulfuric acid solution in an amount of slightly greater than the stoichiometric amount required for the decomposition of the oil lees according to the usual method and then the system was boiled by injecting steam for 2 hours. After sufficiently allowing the system to stand, only the upper oil phase was recovered and the latter was sufficiently washed with water to thus give 928 g of an oil and fat composition.

The resulting oil and fat composition was inspected for the contents of oleanolic acid and maslinic acid present therein according to the capillary GC technique. As a result, the oleanolic acid and maslinic acid contents were found to be 5.1% and 0.8%, respectively.

**Table 1**

| Example No. | 1 | 2 | 3 |
|---|---|---|---|
| Raw Material | Extraction residue 1kg | Extraction residue 1kg | Extraction residue 1kg |
| Extraction solvent | Water-containing ethanol | Ethanol | Ethyl acetate |

| Oil and Fat Composition | | | |
|---|---|---|---|
| Mass | 52 g | 35.8 g | 37.4 g |
| Content of oleanolic acid | 5.2% (2.7 g) | 7.1% (2.5 g) | 7.3% (2.7 g) |
| Content of maslinic acid | 12.9% (6.7 g) | 16.1% (5.8 g) | 11.0% (4.1 g) |
| Content of oil fraction | 20.4% (10.6 g) | 55.9% (20.0 g) | 60.2% (22.5 g) |

| Concentrated Oil and Fat Composition | | | |
|---|---|---|---|
| Mass | 25.2 | -- | -- |
| Content of oleanolic acid | 10.3% (2.6 g) | -- | -- |
| Content of maslinic acid | 26.1% (6.6 g) | -- | -- |
| Content of oil fraction | 41.7% (10.5 g) | -- | -- |

The results obtained in Examples 1 to 3 indicate that the method of the present invention permits the preparation of an oil and fat composition comprising oleanolic acid and maslinic acid.

First of all, the foregoing results indicate that regarding the amounts (masses) of the resulting oil and fat compositions, the best result was obtained when water- containing ethanol as a water-containing organic solvent was used and that regarding the oil fraction content in the composition, the best result was obtained when ethyl acetate as a hydrophobic solvent was used.

Moreover, regarding the contents (masses) of oleanolic acid and maslinic acid present in the resulting oil and fat compositions, the yield (content) of oleanolic acid was excellent in cases wherein ethyl acetate and water-containing ethanol were used as extraction solvents, while the yield (content) of maslinic acid was most excellent in the case of the extraction with water-containing ethanol and the second highest result was obtained when the extraction was carried out using ethanol. Therefore, the extraction with water-containing ethanol is considered to be quite excellent since both of oleanolic acid and maslinic acid can be obtained in higher yields.

Moreover, the concentrated oil and fat composition. obtained by concentrating the oil and fat composition obtained through the extraction with water-containing ethanol permits the improvement of not only the content of the oil fraction, but also the contents of oleanolic acid and maslinic acid, although the former is prepared by a quite simple method. Thus, the method of the invention permits the preparation of such a concentrated oil and fat composition and therefore, the method is quite excellent technique.

The method of the present invention permits the production of oil and fat compositions containing oleanolic acid and maslinic acid and physiologically acceptable salts thereof or derivatives thereof, starting from olive plants and/or by-products obtained in the olive oil-manufacturing process and the method further permits the industrially efficient preparation of oil and fat compositions having higher contents of oleanolic acid and maslinic acid and physiologically acceptable salts thereof or derivatives thereof.

These compositions can be prepared from the by-products obtained in the olive oil-manufacturing process and therefore, the method of the invention is industrially quite excellent from the viewpoint of the production cost.

## Claims

1. A method for preparing an oil and fat composition, which contains at least one member selected from the group consisting of oleanolic acid, maslinic acid, physiologically acceptable salts thereof and derivatives thereof, wherein the method comprises the step of extracting olive plants and/or a by-product obtained in an olive oil-manufacturing process with an organic solvent or a water-containing organic solvent.

2. A method for preparing an oil and fat composition, which contains at least one member selected from the group consisting of oleanolic acid, maslinic acid and physiologically acceptable salts thereof, wherein the method comprises the step of extracting olive plants and/or a by-product obtained in an olive oil-manufacturing process with an organic solvent or a water-containing organic solvent.

3. The method of claim 1 or 2, wherein the organic solvent or water-containing organic solvent is ethyl alcohol, a water-containing ethyl alcohol having an alcohol content ranging from 30 to 95% by mass, ethyl acetate or hexane.

4. The method of claim 1 or 2, wherein the by-product obtained in the olive oil-manufacturing process is a member selected from the group consisting of compression residues, extraction residues, filtered products, deodorization scum, wax components, deacidified oil lees and dark oil.

5. A method for preparing an oil and fat composition, which contains at least one member selected from the group consisting of oleanolic acid, maslinic acid, physiologically acceptable salts thereof and derivatives thereof, wherein the method comprises the step of extracting olive plants and/or a by-product obtained in an olive oil-manufacturing process with an organic solvent or a water-containing organic solvent to obtain an oil and fat composition having an oil fraction content ranging from 20 to 60% by mass.

6. The preparation method of claim 5, wherein the by-product obtained in the olive oil-manufacturing process is a member selected from the group consisting of compression residues, extraction residues, filtered products, deodorization scum, wax components, deacidified oil lees and dark oil.

7. The preparation method of claim 5, wherein the by-product obtained in the olive oil-manufacturing process is extracted with ethyl alcohol, a water-containing ethyl alcohol having an alcohol content ranging from 30 to 95% by mass, ethyl acetate or hexane.

8. The preparation method of claim 5, wherein the resulting oil and fat composition comprises oleanolic acid and maslinic acid.

9. A method for preparing an oil and fat composition, which contains at least one member selected from the group consisting of oleanolic acid, maslinic acid, physiologically acceptable salts thereof and derivatives thereof, wherein the method comprises the step of
(a) extracting olive plants and/or a by-product obtained in an olive oil-manufacturing process with an organic solvent or a water-containing organic solvent, and
(b) treating the resulting extract with a weak alkali.

10. The preparation method of claim 9, wherein the weak alkali is a salt of a weak acid with a strong alkali or ammonia.

11. The preparation method of claim 9, wherein the by-product obtained in the olive oil-manufacturing process is a member selected from the group consisting of compression residues, extraction residues, filtered products, deodorization scum, wax components, deacidified oil lees and dark oil.

12. The preparation method of claim 9, wherein the organic solvent or water- containing organic solvent is ethyl alcohol, a water-containing ethyl alcohol having an alcohol content ranging from 30 to 95% by mass, ethyl acetate or hexane.

13. The preparation method of claim 9, wherein the content of the oil fraction present in the oil and fat composition prepared in the step (a) ranges from 20 to 60% by mass.

14. The preparation method of claim 9, wherein the resulting oil and fat composition comprises oleanolic acid and maslinic acid.

15. A method for preparing an oil and fat composition, which contains at least one member selected from the group consisting of oleanolic acid, maslinic acid, physiologically acceptable salts thereof and derivatives thereof, wherein the method comprises the step of
(a) extracting olive plants and/or a by-product obtained in an olive oil-manufacturing process with an organic solvent or a water-containing organic solvent, and
(c) subjecting the resulting extract to a light steam-distillation treatment.

16. The preparation method of claim 15, wherein the light steam-distillation treatment is carried out at a degree of vacuum ranging from 1.33 × 10² to 1.33 × 10³ Pa.

17. The preparation method of claim 15, wherein the by-product obtained in the olive oil-manufacturing process is a member selected from the group consisting of compression residues, extraction residues, filtered products, deodorization scum, wax components, deacidified oil lees and dark oil.

18. The preparation method of claim 15, wherein the organic solvent or water-containing organic solvent is ethyl alcohol, a water-containing ethyl alcohol having an alcohol content ranging from 30 to 95% by mass, ethyl acetate or hexane.

19. The preparation method of claim 15, wherein the content of the oil fraction present in the oil and fat composition prepared in the step (a) ranges from 20 to 60% by mass.

20. The preparation method of claim 15, wherein the resulting oil and fat composition comprises oleanolic acid and maslinic acid.
